# EUROPEAN PATENT APPLICATION

(11) **EP 1 950 209 A1**
(43) Date of publication of application: **30.07.2008**
(21) Application number: 06812121.9
(22) Date of filing: 23.10.2006
(51) Int. Cl.: C07D 311/56, A61K 31/37, A61P 7/02, A61P 9/10

(54) **AGENT FOR TREATMENT OF CIRCULATORY FAILURE**

(30) Priority: 25.10.2005 JP 2005309771
(71) Applicant: ACTIVUS PHARMA CO., LTD., Yotsukaido-shi, Chiba 284-0008 (JP)
(72) Inventor: TAKAGAKI, Hidetsugu, c/o DIC Corporation, Sakura-shi Chiba 285-8668 (JP); AOKI, Yasuo, Yotsukaido-shi Chiba 284-0027 (JP); ISHIWARA, Mitsuteru, c/o DIC Corporation, Sakura-shi Chiba 285-8668 (JP)
(74) Representative: Albrecht, Thomas
(86) International application number: PCT/JP2006/321052
(87) International publication number: WO 2007/049553

(57) **Abstract**

The present invention relate to a drug for treating circulatory insufficiency containing a benzopyran derivative represented by the following general formula (I): 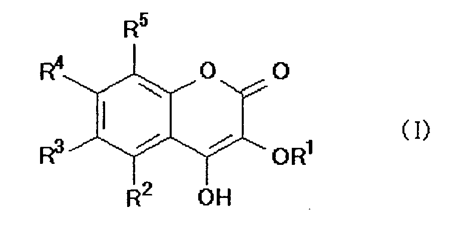 and/or a physiologically acceptable salt thereof as an active ingredient, wherein R¹ is an alkyl group having 1 to 10 carbon atoms, or an alkenyl group having 2 to 10 carbon atoms; and any one of R², R³, R⁴ and R⁵ is a hydroxyl group, an alkoxy group, an alkenyloxy group, an alkoxy group substituted with a hydroxyl group, or an alkoxy group substituted with a carboxy group, and the others are hydrogen atoms.

## Description

### TECHNICAL FIELD

The present invention relates to a drug for treating circulatory insufficiency containing a benzopyran derivative and/or a physiologically acceptable salt thereof as an active ingredient.

### BACKGROUND ART

An anti-allergy agent containing as an active ingredient a benzopyran derivative represented by the following general formula is known: 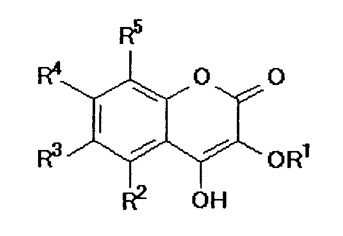 (wherein R¹ is an alkyl group having 1 to 10 carbon atoms or an alkenyl group having 2 to 10 carbon atoms; and any one of R², R³, R⁴ and R⁵ is an alkoxy group substituted with a hydroxyl group or an alkoxy group substituted with a carboxy group, and the others are hydrogen atoms) (see Patent Publication No.1).

There is also a publication disclosing an agent for treating heart disease containing as an active ingredient a benzopyran derivative represented by the following general formula: 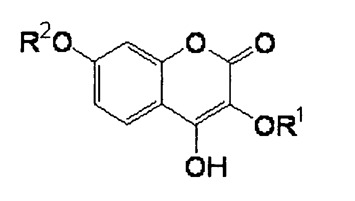 (wherein R¹ is an alkyl group or an alkenyl group; and R² is a hydrogen atom, an alkyl group, an alkyl group having a hydroxyl group, an alkenyl group, an acyl group or a glycosyl group) (see Patent Publication No.2).

However, there was no a suggestion or a teaching at all in either Patent Publication No.1 or No.2 that the benzopyran derivatives could be effective in the treatment of circulatory insufficiency and be extremely useful drugs for treating circulatory insufficiency.

Furthermore, it has been disclosed that the benzopyran derivatives represented by the following formulas have a platelet anti-aggregating effect, and are useful for treating or preventing thrombosis (for example, see Non-patent Publication No.1 and Patent Publication No.3).

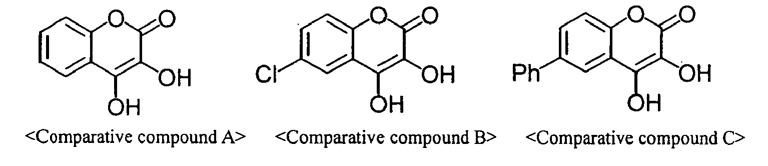

However, there was no specific description with regard to stability or bioabsorption of these compounds in these publications, and whether these compounds had favorable characteristics as pharmaceutical agents had been totally unknown.

Additionally, aspirin cilostazol, beraprost sodium, ticlopidine hydrochloride, among others have been used as an antiplatelet drug, anticoagulant drug or the like (for example, see Non-patent Publication No.2). However, the use of aspirin cilostazol, beraprost sodium, or ticlopidine hydrochloride for treatment of peripheral circulation insufficiency exhibits a bleeding tendency as a side effect derived from its antithrombotic effect. Therefore, such drugs are contraindicated in patients with haemorrhage, potential haemorrhage, congestive heart failure, serious haemological abnormality, or serious hepatopathy, or postoperative patients.
Patent Document 1: Japanese Unexamined Patent Application, Publication No.2003-81827
Patent Document 2: Japanese Unexamined Patent Application, Publication No. Hei 9-315967
Patent Document 3: US Patent No.4845121
Non-patent Document1: Donald. T. Witiak, J. Med. Chem., vol.31, p.1437-1445, 1988
Non-patent Document 2: Kyou no chiryouyaku (Today's medicine), Nankodo, P.476-478, 2002

### DISCLOSURE OF INVENTION

The object of the present invention is to provide very useful drugs for treating circulatory insufficiency which have excellent safety, stability and absorption, and which have an extremely low haemorrhagic adverse reaction, and which are effective in treatment of circulatory insufficiency.

In order to achieve the object of the present invention described above, the inventors synthesized numerous types of compounds and evaluated these for their effectiveness in improving circulatory insufficiency and their safety, stability, absorption and bleeding effect, whereupon they discovered that the benzopyran derivatives shown by the above-mentioned general formula (I) were extremely effective in treating circulatory insufficiency. Specifically, they discovered that the benzopyran derivatives had excellent characteristics such as excellent improving effects in circulatory insufficiency, and that the benzopyran derivatives had superior safety, stability and absorption, compared to the existing drugs, and that the benzopyran derivatives had an extremely low haemorrhagic adverse reaction.

In other words, the present invention provides a drug for treating circulatory insufficiency containing a benzopyran derivative represented by the following general formula (I): 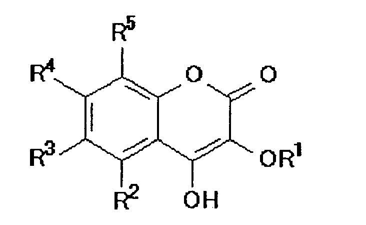 and/or a physiologically acceptable salt thereof as an active ingredient, wherein R¹ is an alkyl group having 1 to 10 carbon atoms, or an alkenyl group having 2 to 10 carbon atoms; and any one of R², R³, R⁴ and R⁵ is a hydroxyl group, an alkoxy group, an alkenyloxy group, an alkoxy group substituted with a hydroxyl group, or an alkoxy group substituted with a carboxy group, and the others are hydrogen atoms.

Moreover, the present invention relates to use of the aforementioned drug for treating circulatory insufficiency.

Furthermore, the present invention also provides a method for treating circulatory insufficiency, the method including: using the aforementioned drug for treating circulatory insufficiency.

The present invention can provide an excellent drug for treating circulatory insufficiency which has high safety, stability and absorption, and which has an extremely low haemorrhagic adverse reaction because a benzopyran derivative represented by the general formula (I) is contained therein as an active ingredient.

Furthermore, according to the present invention, the use of the aforementioned drug for treating circulatory insufficiency enables effective and safe treatment for circulatory insufficiency without causing a haemorrhagic side effect.

Additionally, according to the aforementioned method for treating circulatory insufficiency, circulatory insufficiency can be effectively and safely treated by using the aforementioned drug for treating circulatory insufficiency without causing a haemorrhagic side effect.

### BEST MODE FOR CARRYING OUT THE INVENTION

In the benzopyran derivative represented by the general formula (I) of the present invention, the alkyl group having 1 to 10 carbon atoms of R¹ can be either a straight-chain alkyl group or a branched alkyl group. Examples of such alkyl groups include a methyl group, ethyl group, propyl group, isopropyl group, n-butyl group, n-pentyl group, 2-ethyl-propyl group, n-hexyl group, 4-methylpentyl group, n-heptyl group, 2-ethylhexyl group, n-octyl group, n-nonyl group and n-decyl group.

And the alkenyl group having 2 to 10 carbon atoms of R¹ can be either a straight-chain or branched alkenyl group. Examples of such alkenyl groups include a vinyl group, 2-propenyl group, 2-butenyl group, prenyl group, octenyl group and geranyl group.

For example, the alkoxy group represented by any one of R², R³, R⁴ and R⁵ in the general formula (I) of the present invention can be an alkoxy group having 1 to 10 carbon atoms. More specific examples of such alkoxy groups include a methoxy group, ethoxy group, propoxy group, butoxy group, pentyloxy group, 2-ethylpropoxy group, hexyloxy group, 4-methylpentyloxy group, heptyloxy group, octyloxy group, 2-ethylhexyloxcy group, nonyloxy group, and decyloxy group. The alkoxy groups having 2 to 8 carbon atoms are particularly preferable among these groups.

Additionally, examples of alkenyloxy groups include a vinyloxy group, 2-propenyloxy group, 2-butenyloxy group, prenyloxy group, octenyloxy group, and geranyloxy group.

For example, the alkoxy group substituted with a hydroxyl group, represented by any one of R², R³, R⁴ or R⁵, may be an alkoxy group having 1 to 10 carbon atoms, preferably having 1 to 4 carbon atoms, which is substituted with a hydroxyl group. More specific examples of such alkoxy groups include a 2-hydroxyethoxy group, 3-hydroxypropoxy group, 4-hydroxybutoxy group, 2,3-dihydroxypropoxy group, and 3,4-dihydroxybutoxy group. The aforementioned alkoxy groups substituted with 1 or 2 hydroxyl groups are particularly preferable among these groups.

For example, the alkoxy group substituted with a carboxy group, represented by any one of R², R³, R⁴ and R⁵, may be an alkoxy group having 1 to 4 carbon atoms substituted with a carboxy group. More specific examples of such alkoxy groups include a carboxymethoxy group, 2-carboxyethoxy group, 3-carboxypropoxy group, and 4-carboxybutoxy group. The alkoxy groups substituted with 1 carboxy group are particularly preferable among these groups.

Production of the benzopyran derivatives represented by the general formula (I) can be achieved by selecting a preferable method, depending on the structure of desired benzopyran derivative is planned on. For example, the benzopyran derivative can be produced by the following method disclosed in Japanese Patent Application, First Publication No.2003-81827. Specifically, the method is conducted as shown in the following reaction path.

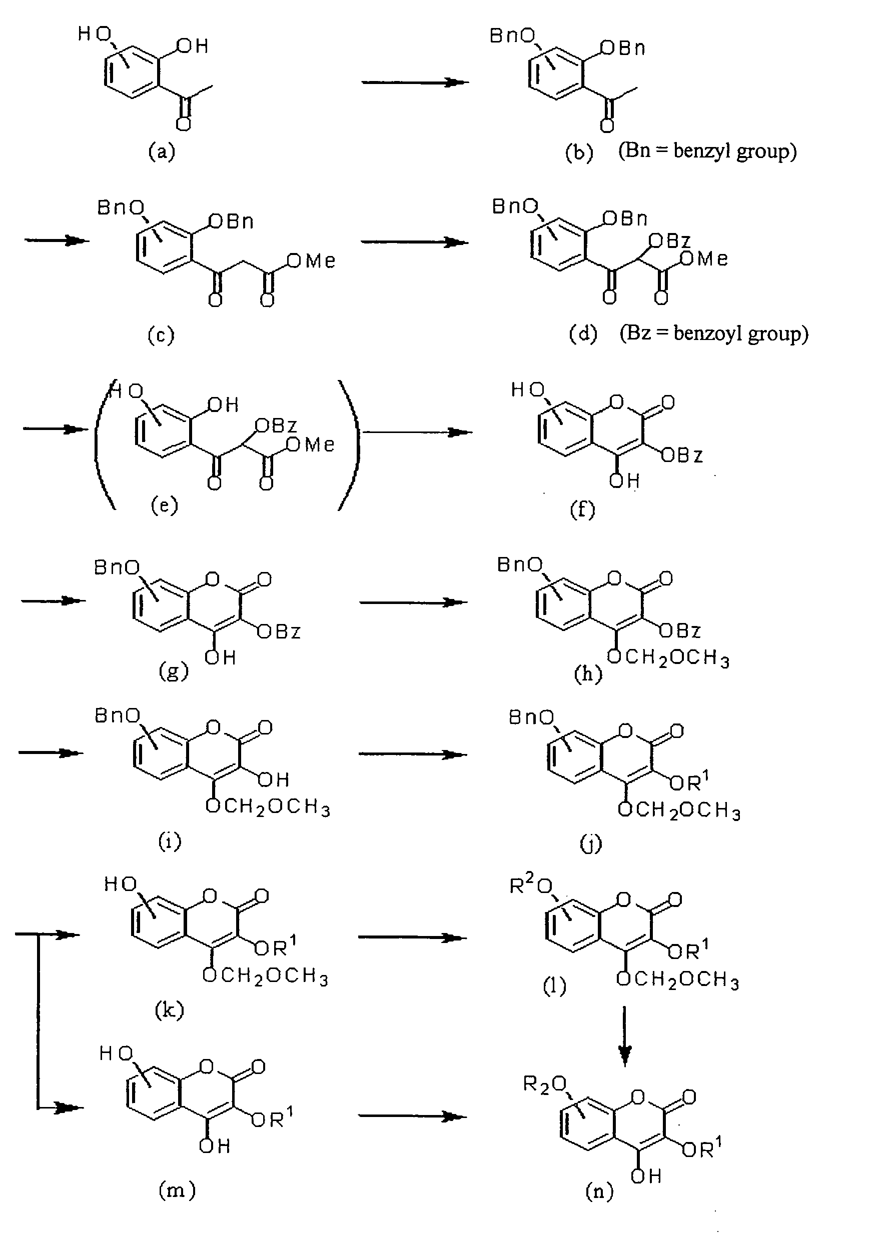

In the reaction path, at first, the hydroxyl groups of dihydroxyacetophenone (a) are protected with a benzyl group to obtain compound (b). Next, a condensation reaction between compound (b) and dimethyl carbonate is carried out to obtain a keto ester compound (c) which is subsequently reacted with benzoyl peroxide to obtain compound (d). At this stage, the benzyl groups used as a protecting group for the hydroxyl group are deprotected by hydrocracking, and then treated with an acid to obtain a benzoyloxy compound (f).

Subsequently, the hydroxyl group on the aromatic ring of this benzoyloxy compound (f) is protected with a benzyl group to obtain compound (g), and then a methoxymethyl group is added to the 4-position to obtain compound (h). After removing the benzoyl group from compound (h), the hydroxyl group at the 3-position is alkylated to obtain compound (j). The alkylation of the hydroxyl group can be performed by a conventional alkylation reaction such as a reaction with an alkyl halide, a sulfate ester, an arylsulfonate ester or the like. Then, the protective group of the hydroxyl group on the aromatic ring is deprotected to obtain compound (k).

In order to obtain the benzopyran derivatives represented by the general formula (I), wherein any one of R², R³, R⁴ and R⁵ is an alkoxy group, an alkenyloxy group, or an alkoxy group substituted with a hydroxyl group or a carboxy group, the hydroxyl group on the aromatic ring of the compound (k) or compound (m) is alkylated with alkylating agents (such as alkyl halide, sulfate ester or arylsulfonate ester); alkenylating agents (such as alkenyl halide, sulfate ester or arylsulfonate ester); or alkylating agents wherein the hydroxyl or carboxy group is protected (such as alkyl halide, sulfate ester or arylsulfonate ester), and then the protected hydroxyl or carboxy group is deprotected.

Furthermore, in order to explain the process of producing the benzopyran derivatives represented by the general formula (I), a method of producing a benzopyran derivative wherein any one of R², R³, R⁴ and R⁵ is a 2-hydroxyethoxy group is specifically explained below.

First, an alkoxylation reaction is performed where 2-acetoxyethyl bromide is reacted with compound (k) in an organic solvent in the presence of a basic compound.

As examples of the basic compounds used in this reaction, there are inorganic salts such as sodium bicarbonate, sodium carbonate, potassium bicarbonate, potassium carbonate, sodium hydroxide and potassium hydroxide; metal alcoholates such as sodium methoxide, sodium ethoxide, sodium t-butoxide and potassium t-butoxide; and metallic hydrides such as sodium hydride and potassium hydride.

Examples of organic solvents used in the reaction include hydrocarbons such as benzene, toluene and xylene; ethers such as diethyl ether, tetrahydrofuran and 1,2-dimethoxyethane; and amides such as N,N-dimethylformamide, N,N-dimethylacetoamide and 1-methyl-2-pyrrolidinone.

The reaction temperature is preferably 0°C to 100°C, and more preferably 20°C to 50°C, and the reaction time is normally 1 to 5 hours.

Next, if necessary, the acetyl group which is a protective group may be removed, and this reaction can be a de-acetylation reaction conducted under ordinary alkaline conditions. In this way, the objective benzopyran derivatives substituted with a 2-hydroxyethoxy group can be produced.

The following compounds are illustrative examples of the benzopyran derivatives of the present invention, represented by the general formula (I), however these examples are intended to illustrate the invention and not to be construed to limit the scope of the invention.

**Table 1**

| Compound No. | R¹ | R² | R³ | R⁴ | R⁵ |
|---|---|---|---|---|---|
| 1 | methyl | hydroxyl | H | H | H |
| 2 | ethyl | hydroxyl | H | H | H |
| 3 | propyl | hydroxyl | H | H | H |
| 4 | isopropyl | hydroxyl | H | H | H |
| 5 | butyl | hydroxyl | H | H | H |
| 6 | s-butyl | hydroxyl | H | H | H |
| 7 | pentyl | hydroxyl | H | H | H |
| 8 | 1-ethylpropyl | hydroxyl | H | H | H |
| 9 | hexyl | hydroxyl | H | H | H |
| 10 | 2-methylpentyl | hydroxyl | H | H | H |
| 11 | heptyl | hydroxyl | H | H | H |
| 12 | 1-ethylpentyl | hydroxyl | H | H | H |
| 13 | 4-methylpentyl | hydroxyl | H | H | H |
| 14 | 4-ethylbutyl | hydroxyl | H | H | H |
| 15 | octyl | hydroxyl | H | H | H |
| 16 | 1-ethylhexyl | hydroxyl | H | H | H |
| 17 | decyl | hydroxyl | H | H | H |
| 18 | vinyl | hydroxyl | H | H | H |
| 19 | 1-propenyl | hydroxyl | H | H | H |
| 20 | 2-butenyl | hydroxyl | H | H | H |
| 21 | 1-hexenyl | hydroxyl | H | H | H |
| 22 | 1-octenyl | hydroxyl | H | H | H |
| 23 | 1-decenyl | hydroxyl | H | H | H |
| 24 | 3-methyl-2-butenyl | hydroxyl | H | H | H |
| 25 | geranyl | hydroxyl | H | H | H |
| 26 | prenyl | hydroxyl | H | H | H |
| 27 | methyl | methoxy | H | H | H |
| 28 | ethyl | methoxy | H | H | H |
| 29 | butyl | methoxy | H | H | H |
| 30 | hexyl | ethoxy | H | H | H |
| 31 | 2-methylpentyl | ethoxy | H | H | H |
| 32 | octyl | ethoxy | H | H | H |
| 33 | decyl | ethoxy | H | H | H |
| 34 | 1-propenyl | isopropoxy | H | H | H |
| 35 | 1-octenyl | isopropoxy | H | H | H |

**Table 2**

| Compound No. | R¹ | R² | R³ | R⁴ | R⁵ |
|---|---|---|---|---|---|
| 36 | geranyl | isopropoxy | H | H | H |
| 37 | ethyl | butoxy | H | H | H |
| 38 | butyl | butoxy | H | H | H |
| 39 | s-butyl | butoxy | H | H | H |
| 40 | hexyl | butoxy | H | H | H |
| 41 | 1-ethylpentyl | hexyloxy | H | H | H |
| 42 | octyl | hexyloxy | H | H | H |
| 43 | 2-butenyl | hexyloxy | H | H | H |
| 44 | prenyl | hexyloxy | H | H | H |
| 45 | ethyl | octyloxy | H | H | H |
| 46 | butyl | octyloxy | H | H | H |
| 47 | hexyl | octyloxy | H | H | H |
| 48 | octyl | octyloxy | H | H | H |
| 49 | decyl | decyloxy | H | H | H |
| 50 | 1-hexenyl | decyloxy | H | H | H |
| 51 | 3-methyl-2-butenyl | decyloxy | H | H | H |
| 52 | methyl | 1-octenyloxy | H | H | H |
| 53 | ethyl | 1-octenyloxy | H | H | H |
| 54 | hexyl | 1-octenyloxy | H | H | H |
| 55 | octyl | 1-octenyloxy | H | H | H |
| 56 | 1-propenyl | 1-octenyloxy | H | H | H |
| 57 | 1-octenyl | 1-octenyloxy | H | H | H |
| 58 | geranyl | geranyloxy | H | H | H |
| 59 | methyl | H | hydroxyl | H | H |
| 60 | ethyl | H | hydroxyl | H | H |
| 61 | propyl | H | hydroxyl | H | H |
| 62 | isopropyl | H | hydroxyl | H | H |
| 63 | butyl | H | hydroxyl | H | H |
| 64 | s-butyl | H | hydroxyl | H | H |
| 65 | pentyl | H | hydroxyl | H | H |
| 66 | 1-ethylpropyl | H | hydroxyl | H | H |
| 67 | hexyl | H | hydroxyl | H | H |
| 68 | 2-methylpentyl | H | hydroxyl | H | H |
| 69 | heptyl | H | hydroxyl | H | H |
| 70 | 1-ethylpentyl | H | hydroxyl | H | H |

**Table 3**

| Compound No. | R¹ | R² | R³ | R⁴ | R⁵ |
|---|---|---|---|---|---|
| 71 | 4-methylpentyl | H | hydroxyl | H | H |
| 72 | 4-ethylbutyl | H | hydroxyl | H | H |
| 73 | octyl | H | hydroxyl | H | H |
| 74 | 1-ethylhexyl | H | hydroxyl | H | H |
| 75 | decyl | H | hydroxyl | H | H |
| 76 | vinyl | H | hydroxyl | H | H |
| 77 | 1-propenyl | H | hydroxyl | H | H |
| 78 | 2-butenyl | H | hydroxyl | H | H |
| 79 | 1-hexenyl | H | hydroxyl | H | H |
| 80 | 1-octenyl | H | hydroxyl | H | H |
| 81 | 1-decenyl | H | hydroxyl | H | H |
| 82 | 3-methyl-2-butenyl | H | hydroxyl | H | H |
| 83 | geranyl | H | hydroxyl | H | H |
| 84 | prenyl | H | hydroxyl | H | H |
| 85 | methyl | H | methoxy | H | H |
| 86 | ethyl | H | methoxy | H | H |
| 87 | butyl | H | methoxy | H | H |
| 88 | hexyl | H | ethoxy | H | H |
| 89 | 2-methylpentyl | H | ethoxy | H | H |
| 90 | octyl | H | ethoxy | H | H |
| 91 | decyl | H | ethoxy | H | H |
| 92 | 1-propenyl | H | isopropoxy | H | H |
| 93 | 1-octenyl | H | isopropoxy | H | H |
| 94 | geranyl | H | isopropoxy | H | H |
| 95 | ethyl | H | butoxy | H | H |
| 96 | butyl | H | butoxy | H | H |
| 97 | s-butyl | H | butoxy | H | H |
| 98 | hexyl | H | butoxy | H | H |
| 99 | 1-ethylpentyl | H | hexyloxy | H | H |
| 100 | octyl | H | hexyloxy | H | H |
| 101 | 2-butenyl | H | hexyloxy | H | H |
| 102 | prenyl | H | hexyloxy | H | H |
| 103 | ethyl | H | octyloxy | H | H |
| 104 | butyl | H | octyloxy | H | H |
| 105 | hexyl | H | octyloxy | H | H |

**Table 4**

| Compound No. | R¹ | R² | R³ | R⁴ | R⁵ |
|---|---|---|---|---|---|
| 106 | octyl | H | octyloxy | H | H |
| 107 | decyl | H | decyloxy | H | H |
| 108 | 1-hexenyl | H | decyloxy | H | H |
| 109 | 3-methyl-2-butenyl | H | decyloxy | H | H |
| 110 | methyl | H | 1-octenyloxy | H | H |
| 111 | ethyl | H | 1-octenyloxy | H | H |
| 112 | hexyl | H | 1-octenyloxy | H | H |
| 113 | octyl | H | 1-octenyloxy | H | H |
| 114 | 1-propenyl | H | 1-octenyloxy | H | H |
| 115 | 1-octenyl | H | 1-octenyloxy | H | H |
| 116 | geranyl | H | geranyloxy | H | H |
| 117 | methyl | H | H | hydroxyl | H |
| 118 | ethyl | H | H | hydroxyl | H |
| 119 | propyl | H | H | hydroxyl | H |
| 120 | isopropyl | H | H | hydroxyl | H |
| 121 | butyl | H | H | hydroxyl | H |
| 122 | s-butyl | H | H | hydroxyl | H |
| 123 | pentyl | H | H | hydroxyl | H |
| 124 | 1-ethylpropyl | H | H | hydroxyl | H |
| 125 | hexyl | H | H | hydroxyl | H |
| 126 | 2-methylpentyl | H | H | hydroxyl | H |
| 127 | heptyl | H | H | hydroxyl | H |
| 128 | 1-ethylpentyl | H | H | hydroxyl | H |
| 129 | 4-methylpentyl | H | H | hydroxyl | H |
| 130 | 4-ethylbutyl | H | H | hydroxyl | H |
| 131 | octyl | H | H | hydroxyl | H |
| 132 | 1-ethylhexyl | H | H | hydroxyl | H |
| 133 | decyl | H | H | hydroxyl | H |
| 134 | vinyl | H | H | hydroxyl | H |
| 135 | 1-propenyl | H | H | hydroxyl | H |
| 136 | 2-butenyl | H | H | hydroxyl | H |
| 137 | 1-hexenyl | H | H | hydroxyl | H |
| 138 | 1-octenyl | H | H | hydroxyl | H |
| 139 | 1-decenyl | H | H | hydroxyl | H |
| 140 | 3-methyl-2-butenyl | H | H | hydroxyl | H |

**Table 5**

| Compound No. | R¹ | R² | R³ | R⁴ | R⁵ |
|---|---|---|---|---|---|
| 141 | geranyl | H | H | hydroxyl | H |
| 142 | prenyl | H | H | hydroxyl | H |
| 143 | methyl | H | H | methoxy | H |
| 144 | ethyl | H | H | methoxy | H |
| 145 | butyl | H | H | methoxy | H |
| 146 | hexyl | H | H | ethoxy | H |
| 147 | 2-methylpentyl | H | H | ethoxy | H |
| 148 | octyl | H | H | ethoxy | H |
| 149 | decyl | H | H | ethoxy | H |
| 150 | 1-propenyl | H | H | isopropoxy | H |
| 151 | 1-octenyl | H | H | isopropoxy | H |
| 152 | geranyl | H | H | isopropoxy | H |
| 153 | ethyl | H | H | butoxy | H |
| 154 | butyl | H | H | butoxy | H |
| 155 | s-butyl | H | H | butoxy | H |
| 156 | hexyl | H | H | butoxy | H |
| 157 | 1-ethylpentyl | H | H | hexyloxy | H |
| 158 | octyl | H | H | hexyloxy | H |
| 159 | 2-butenyl | H | H | hexyloxy | H |
| 160 | prenyl | H | H | hexyloxy | H |
| 161 | ethyl | H | H | octyloxy | H |
| 162 | butyl | H | H | octyloxy | H |
| 163 | hexyl | H | H | octyloxy | H |
| 164 | octyl | H | H | octyloxy | H |
| 165 | decyl | H | H | decyloxy | H |
| 166 | 1-hexenyl | H | H | decyloxy | H |
| 167 | 3-methyl-2-butenyl | H | H | decyloxy | H |
| 168 | methyl | H | H | 1-octenyloxy | H |
| 169 | ethyl | H | H | 1-octenyloxy | H |
| 170 | hexyl | H | H | 1-octenyloxy | H |
| 171 | octyl | H | H | 1-octenyloxy | H |
| 172 | 1-propenyl | H | H | 1-octenyloxy | H |
| 173 | 1-octenyl | H | H | 1-octenyloxy | H |
| 174 | geranyl | H | H | geranyloxy | H |
| 175 | methyl | H | H | H | hydroxyl |

**Table 6**

| Compound No. | R¹ | R² | R³ | R⁴ | R⁵ |
|---|---|---|---|---|---|
| 176 | ethyl | H | H | H | hydroxyl |
| 177 | propyl | H | H | H | hydroxyl |
| 178 | isopropyl | H | H | H | hydroxyl |
| 179 | butyl | H | H | H | hydroxyl |
| 180 | s-butyl | H | H | H | hydroxyl |
| 181 | pentyl | H | H | H | hydroxyl |
| 182 | 1-ethylpropyl | H | H | H | hydroxyl |
| 183 | hexyl | H | H | H | hydroxyl |
| 184 | 2-methylpentyl | H | H | H | hydroxyl |
| 185 | heptyl | H | H | H | hydroxyl |
| 186 | 1-ethylpentyl | H | H | H | hydroxyl |
| 187 | 4-methylpentyl | H | H | H | hydroxyl |
| 188 | 4-ethylbutyl | H | H | H | hydroxyl |
| 189 | octyl | H | H | H | hydroxyl |
| 190 | 1-ethylhexyl | H | H | H | hydroxyl |
| 191 | decyl | H | H | H | hydroxyl |
| 192 | vinyl | H | H | H | hydroxyl |
| 193 | 1-propenyl | H | H | H | hydroxyl |
| 194 | 2-butenyl | H | H | H | hydroxyl |
| 195 | 1-hexenyl | H | H | H | hydroxyl |
| 196 | 1-octenyl | H | H | H | hydroxyl |
| 197 | 1-decenyl | H | H | H | hydroxyl |
| 198 | 3-methyl-2-butenyl | H | H | H | hydroxyl |
| 199 | geranyl | H | H | H | hydroxyl |
| 200 | prenyl | H | H | H | hydroxyl |
| 201 | methyl | H | H | H | methoxy |
| 202 | ethyl | H | H | H | methoxy |
| 203 | butyl | H | H | H | methoxy |
| 204 | hexyl | H | H | H | ethoxy |
| 205 | 2-methylpentyl | H | H | H | ethoxy |
| 206 | octyl | H | H | H | ethoxy |
| 207 | decyl | H | H | H | ethoxy |
| 208 | 1-propenyl | H | H | H | isopropoxy |
| 209 | 1-octenyl | H | H | H | isopropoxy |
| 210 | geranyl | H | H | H | isopropoxy |

**Table 7**

| Compound No. | R¹ | R² | R³ | R⁴ | R⁵ |
|---|---|---|---|---|---|
| 211 | ethyl | H | H | H | butoxy |
| 212 | butyl | H | H | H | butoxy |
| 213 | s-butyl | H | H | H | butoxy |
| 214 | hexyl | H | H | H | butoxy |
| 215 | 1-ethylpentyl | H | H | H | hexyloxy |
| 216 | octyl | H | H | H | hexyloxy |
| 217 | 2-butenyl | H | H | H | hexyloxy |
| 218 | prenyl | H | H | H | hexyloxy |
| 219 | ethyl | H | H | H | octyloxy |
| 220 | butyl | H | H | H | octyloxy |
| 221 | hexyl | H | H | H | octyloxy |
| 222 | octyl | H | H | H | octyloxy |
| 223 | decyl | H | H | H | decyloxy |
| 224 | 1-hexenyl | H | H | H | decyloxy |
| 225 | 3-methyl-2-butenyl | H | H | H | decyloxy |
| 226 | methyl | H | H | H | 1-octenyloxy |
| 227 | ethyl | H | H | H | 1-octenyloxy |
| 228 | hexyl | H | H | H | 1-octenyloxy |
| 229 | octyl | H | H | H | 1-octenyloxy |
| 230 | 1-propenyl | H | H | H | 1-octenyloxy |
| 231 | 1-octenyl | H | H | H | 1-octenyloxy |
| 232 | geranyl | H | H | H | geranyloxy |
| 233 | methyl | 2-hydroxyethoxy | H | H | H |
| 234 | ethyl | 2-hydroxyethoxy | H | H | H |
| 235 | propyl | 2-hydroxyethoxy | H | H | H |
| 236 | isopropyl | 2-hydroxyethoxy | H | H | H |
| 237 | butyl | 2-hydroxyethoxy | H | H | H |
| 238 | s-butyl | 2-hydroxyethoxy | H | H | H |
| 239 | pentyl | 2-hydroxyethoxy | H | H | H |
| 240 | 1-ethylpropyl | 2-hydroxyethoxy | H | H | H |
| 241 | hexyl | 2-hydroxyethoxy | H | H | H |
| 242 | 2-methylpentyl | 2-hydroxyethoxy | H | H | H |
| 243 | heptyl | 2-hydroxyethoxy | H | H | H |
| 244 | 1-ethylpentyl | 2-hydroxyethoxy | H | H | H |
| 245 | 4-methylpentyl | 2-hydroxyethoxy | H | H | H |

**Table 8**

| Compound No. | R¹ | R² | R³ | R⁴ | R⁵ |
|---|---|---|---|---|---|
| 246 | 4-ethylbutyl | 2-hydroxyethoxy | H | H | H |
| 247 | octyl | 2-hydroxyethoxy | H | H | H |
| 248 | 1-ethylhexyl | 1-hydroxymethoxy | H | H | H |
| 249 | decyl | 1-hydroxymethoxy | H | H | H |
| 250 | vinyl | 1-hydroxymethoxy | H | H | H |
| 251 | 1-propenyl | 1-hydroxymethoxy | H | H | H |
| 252 | 2-butenyl | 1-hydroxymethoxy | H | H | H |
| 253 | 1-hexenyl | 1-hydroxymethoxy | H | H | H |
| 254 | 1-octenyl | 1-hydroxymethoxy | H | H | H |
| 255 | 1-decenyl | 1-hydroxymethoxy | H | H | H |
| 256 | 3-methyl-2-butenyl | 1-hydroxymethoxy | H | H | H |
| 257 | geranyl | 1-hydroxymethoxy | H | H | H |
| 258 | prenyl | 1-hydroxymethoxy | H | H | H |
| 259 | methyl | 3-hydroxypropoxy | H | H | H |
| 260 | ethyl | 3-hydroxypropoxy | H | H | H |
| 261 | butyl | 3-hydroxypropoxy | H | H | H |
| 262 | hexyl | 3-hydroxypropoxy | H | H | H |
| 263 | 2-methylpentyl | 3-hydroxypropoxy | H | H | H |
| 264 | octyl | 3-hydroxypropoxy | H | H | H |
| 265 | decyl | 3-hydroxypropoxy | H | H | H |
| 266 | 1-propenyl | 3-hydroxypropoxy | H | H | H |
| 267 | 1-octenyl | 3-hydroxypropoxy | H | H | H |
| 268 | geranyl | 3-hydroxypropoxy | H | H | H |
| 269 | ethyl | 4-hydroxybutoxy | H | H | H |
| 270 | butyl | 4-hydroxybutoxy | H | H | H |
| 271 | s-butyl | 4-hydroxybutoxy | H | H | H |
| 272 | hexyl | 4-hydroxybutoxy | H | H | H |
| 273 | 1-ethylpentyl | 4-hydroxybutoxy | H | H | H |
| 274 | octyl | 4-hydroxybutoxy | H | H | H |
| 275 | 2-butenyl | 4-hydroxybutoxy | H | H | H |
| 276 | prenyl | 4-hydroxybutoxy | H | H | H |
| 277 | ethyl | 2,3-dihydroxypropoxy | H | H | H |
| 278 | butyl | 2,3-dihydroxypropoxy | H | H | H |
| 279 | hexyl | 2,3-dihydroxypropoxy | H | H | H |
| 280 | octyl | 2,3-dihydroxypropoxy | H | H | H |

**Table 9**

| Compound No. | R¹ | R² | R³ | R⁴ | R⁵ |
|---|---|---|---|---|---|
| 281 | decyl | 2,3-dihydroxypropoxy | H | H | H |
| 282 | 1-hexenyl | 2,3-dihydroxypropoxy | H | H | H |
| 283 | 3-methyl-2-butenyl | 2,3-dihydroxypropoxy | H | H | H |
| 284 | methyl | 3,4-dihydroxybutoxy | H | H | H |
| 285 | ethyl | 3,4-dihydroxybutoxy | H | H | H |
| 286 | hexyl | 3,4-dihydroxybutoxy | H | H | H |
| 287 | octyl | 3,4-dihydroxybutoxy | H | H | H |
| 288 | 1-propenyl | 3,4-dihydroxybutoxy | H | H | H |
| 289 | 1-octenyl | 3,4-dihydroxybutoxy | H | H | H |
| 290 | geranyl | 3,4-dihydroxybutoxy | H | H | H |
| 291 | methyl | carboxymethoxy | H | H | H |
| 292 | ethyl | carboxymethoxy | H | H | H |
| 293 | propyl | carboxymethoxy | H | H | H |
| 294 | isopropyl | carboxymethoxy | H | H | H |
| 295 | butyl | carboxymethoxy | H | H | H |
| 296 | s-butyl | carboxymethoxy | H | H | H |
| 297 | pentyl | carboxymethoxy | H | H | H |
| 298 | hexyl | carboxymethoxy | H | H | H |
| 299 | 2-methylpentyl | carboxymethoxy | H | H | H |
| 300 | heptyl | carboxymethoxy | H | H | H |
| 301 | 1-ethylpentyl | carboxymethoxy | H | H | H |
| 302 | 4-methylpentyl | carboxymethoxy | H | H | H |
| 303 | 1-ethylhexyl | carboxymethoxy | H | H | H |
| 304 | octyl | carboxymethoxy | H | H | H |
| 305 | 1-ethylhexyl | carboxymethoxy | H | H | H |
| 306 | decyl | carboxymethoxy | H | H | H |
| 307 | vinyl | carboxymethoxy | H | H | H |
| 308 | 1-propenyl | carboxymethoxy | H | H | H |
| 309 | 2-butenyl | carboxymethoxy | H | H | H |
| 310 | 1-hexenyl | carboxymethoxy | H | H | H |
| 311 | 1-octenyl | carboxymethoxy | H | H | H |
| 312 | 1-decenyl | carboxymethoxy | H | H | H |
| 313 | 3-methyl-2-butenyl | carboxymethoxy | H | H | H |
| 314 | geranyl | carboxymethoxy | H | H | H |
| 315 | prenyl | carboxymethoxy | H | H | H |

**Table 10**

| Compound No. | R¹ | R² | R³ | R⁴ | R⁵ |
|---|---|---|---|---|---|
| 316 | methyl | 2-carboxyethoxy | H | H | H |
| 317 | ethyl | 2-carboxyethoxy | H | H | H |
| 318 | butyl | 2-carboxyethoxy | H | H | H |
| 319 | hexyl | 2-carboxyethoxy | H | H | H |
| 320 | octyl | 2-carboxyethoxy | H | H | H |
| 321 | 1-propenyl | 2-carboxyethoxy | H | H | H |
| 322 | 1-octenyl | 2-carboxyethoxy | H | H | H |
| 323 | geranyl | 2-carboxyethoxy | H | H | H |
| 324 | ethyl | 3-carboxypropoxy | H | H | H |
| 325 | butyl | 3-carboxypropoxy | H | H | H |
| 326 | hexyl | 3-carboxypropoxy | H | H | H |
| 327 | octyl | 3-carboxypropoxy | H | H | H |
| 328 | 2-butenyl | 3-carboxypropoxy | H | H | H |
| 329 | prenyl | 3-carboxypropoxy | H | H | H |
| 330 | ethyl | 4-carboxybutoxy | H | H | H |
| 331 | butyl | 4-carboxybutoxy | H | H | H |
| 332 | hexyl | 4-carboxybutoxy | H | H | H |
| 333 | octyl | 4-carboxybutoxy | H | H | H |
| 334 | 1-octenyl | 4-carboxybutoxy | H | H | H |
| 335 | methyl | H | 2-hydroxyethoxy | H | H |
| 336 | ethyl | H | 2-hydroxyethoxy | H | H |
| 337 | propyl | H | 2-hydroxyethoxy | H | H |
| 338 | isopropyl | H | 2-hydroxyethoxy | H | H |
| 339 | butyl | H | 2-hydroxyethoxy | H | H |
| 340 | s-butyl | H | 2-hydroxyethoxy | H | H |
| 341 | pentyl | H | 2-hydroxyethoxy | H | H |
| 342 | 1-ethylpropyl | H | 2-hydroxyethoxy | H | H |
| 343 | hexyl | H | 2-hydroxyethoxy | H | H |
| 344 | 2-methylpentyl | H | 2-hydroxyethoxy | H | H |
| 345 | heptyl | H | 2-hydroxyethoxy | H | H |
| 346 | 1-ethylpentyl | H | 2-hydroxyethoxy | H | H |
| 347 | 4-methylpentyl | H | 2-hydroxyethoxy | H | H |
| 348 | 1-ethylhexyl | H | 2-hydroxyethoxy | H | H |
| 349 | octyl | H | 2-hydroxyethoxy | H | H |
| 350 | 1-ethylhexyl | H | 1-hydroxymethoxy | H | H |

**Table 11**

| Compound No. | R¹ | R² | R³ | R⁴ | R⁵ |
|---|---|---|---|---|---|
| 351 | decyl | H | 1-hydroxymethoxy | H | H |
| 352 | vinyl | H | 1-hydroxymethoxy | H | H |
| 353 | 1-propenyl | H | 1-hydroxymethoxy | H | H |
| 354 | 2-butenyl | H | 1-hydroxymethoxy | H | H |
| 355 | 1-hexenyl | H | 1-hydroxymethoxy | H | H |
| 356 | 1-octenyl | H | 1-hydroxymethoxy | H | H |
| 357 | 1-decenyl | H | 1-hydroxymethoxy | H | H |
| 358 | 3-methyl2-butenyl | H | 1-hydroxymethoxy | H | H |
| 359 | geranyl | H | 1-hydroxymethoxy | H | H |
| 360 | prenyl | H | 1-hydroxymethoxy | H | H |
| 361 | methyl | H | 3-hydroxypropoxy | H | H |
| 362 | ethyl | H | 3-hydroxypropoxy | H | H |
| 363 | butyl | H | 3-hydroxypropoxy | H | H |
| 364 | hexyl | H | 3-hydroxypropoxy | H | H |
| 365 | 2-methylpentyl | H | 3-hydroxypropoxy | H | H |
| 366 | octyl | H | 3-hydroxypropoxy | H | H |
| 367 | decyl | H | 3-hydroxypropoxy | H | H |
| 368 | 1-propenyl | H | 3-hydroxypropoxy | H | H |
| 369 | 1-octenyl | H | 3-hydroxypropoxy | H | H |
| 370 | geranyl | H | 3-hydroxypropoxy | H | H |
| 371 | ethyl | H | 4-hydroxybutoxy | H | H |
| 372 | butyl | H | 4-hydroxybutoxy | H | H |
| 373 | s-butyl | H | 4-hydroxybutoxy | H | H |
| 374 | hexyl | H | 4-hydroxybutoxy | H | H |
| 375 | 1-ethylpentyl | H | 4-hydroxybutoxy | H | H |
| 376 | octyl | H | 4-hydroxybutoxy | H | H |
| 377 | 2-butenyl | H | 4-hydroxybutoxy | H | H |
| 378 | prenyl | H | 4-hydroxybutoxy | H | H |
| 379 | ethyl | H | 2,3-dihydroxypropoxy | H | H |
| 380 | butyl | H | 2,3-dihydroxypropoxy | H | H |
| 381 | hexyl | H | 2,3-dihydroxypropoxy | H | H |
| 382 | octyl | H | 2,3-dihydroxypropoxy | H | H |
| 383 | decyl | H | 2,3-dihydroxypropoxy | H | H |
| 384 | 1-hexenyl | H | 2,3-dihydroxypropoxy | H | H |
| 385 | 3-methyl-2-butenyl | H | 2,3-dihydroxypropoxy | H | H |

**Table 12**

| Compound No. | R¹ | R² | R³ | R⁴ | R⁵ |
|---|---|---|---|---|---|
| 386 | methyl | H | 3,4-dihydroxybutoxy | H | H |
| 387 | ethyl | H | 3,4-dihydroxybutoxy | H | H |
| 388 | hexyl | H | 3,4-dihydroxybutoxy | H | H |
| 389 | octyl | H | 3,4-dihydroxybutoxy | H | H |
| 390 | 1-propenyl | H | 3,4-dihydroxybutoxy | H | H |
| 391 | 1-octenyl | H | 3,4-dihydroxybutoxy | H | H |
| 392 | geranyl | H | 3,4-dihydroxybutoxy | H | H |
| 393 | methyl | H | carboxymethoxy | H | H |
| 394 | ethyl | H | carboxymethoxy | H | H |
| 395 | propyl | H | carboxymethoxy | H | H |
| 396 | isopropyl | H | carboxymethoxy | H | H |
| 397 | butyl | H | carboxymethoxy | H | H |
| 398 | s-butyl | H | carboxymethoxy | H | H |
| 399 | pentyl | H | carboxymethoxy | H | H |
| 400 | hexyl | H | carboxymethoxy | H | H |
| 401 | 2-methylpentyl | H | carboxymethoxy | H | H |
| 402 | heptyl | H | carboxymethoxy | H | H |
| 403 | 1-ethylpentyl | H | carboxymethoxy | H | H |
| 404 | 4-methylpentyl | H | carboxymethoxy | H | H |
| 405 | 1-ethylhexyl | H | carboxymethoxy | H | H |
| 406 | octyl | H | carboxymethoxy | H | H |
| 407 | 1-ethylhexyl | H | carboxymethoxy | H | H |
| 408 | decyl | H | carboxymethoxy | H | H |
| 409 | vinyl | H | carboxymethoxy | H | H |
| 410 | 1-propenyl | H | carboxymethoxy | H | H |
| 411 | 2-butenyl | H | carboxymethoxy | H | H |
| 412 | 1-hexenyl | H | carboxymethoxy | H | H |
| 413 | 1-octenyl | H | carboxymethoxy | H | H |
| 414 | 1-decenyl | H | carboxymethoxy | H | H |
| 415 | 3-methyl-2-butenyl | H | carboxymethoxy | H | H |
| 416 | geranyl | H | carboxymethoxy | H | H |
| 417 | prenyl | H | carboxymethoxy | H | H |
| 418 | methyl | H | 2-carboxyethoxy | H | H |
| 419 | ethyl | H | 2-carboxyethoxy | H | H |
| 420 | butyl | H | 2-carboxyethoxy | H | H |

**Table 13**

| Compound No. | R¹ | R² | R³ | R⁴ | R⁵ |
|---|---|---|---|---|---|
| 421 | hexyl | H | 2-carboxyethoxy | H | H |
| 422 | octyl | H | 2-carboxyethoxy | H | H |
| 423 | 1-propenyl | H | 2-carboxyethoxy | H | H |
| 424 | 1-octenyl | H | 2-carboxyethoxy | H | H |
| 425 | geranyl | H | 2-carboxyethoxy | H | H |
| 426 | ethyl | H | 3-carboxypropoxy | H | H |
| 427 | butyl | H | 3-carboxypropoxy | H | H |
| 428 | hexyl | H | 3-carboxypropoxy | H | H |
| 429 | octyl | H | 3-carboxypropoxy | H | H |
| 430 | 2-butenyl | H | 3-carboxypropoxy | H | H |
| 431 | prenyl | H | 3-carboxypropoxy | H | H |
| 432 | ethyl | H | 4-carboxybutoxy | H | H |
| 433 | butyl | H | 4-carboxybutoxy | H | H |
| 434 | hexyl | H | 4-carboxybutoxy | H | H |
| 435 | octyl | H | 4-carboxybutoxy | H | H |
| 436 | 1-octenyl | H | 4-carboxybutoxy | H | H |
| 437 | methyl | H | H | 2-hydroxyethoxy | H |
| 438 | ethyl | H | H | 2-hydroxyethoxy | H |
| 439 | propyl | H | H | 2-hydroxyethoxy | H |
| 440 | isopropyl | H | H | 2-hydroxyethoxy | H |
| 441 | butyl | H | H | 2-hydroxyethoxy | H |
| 442 | s-butyl | H | H | 2-hydroxyethoxy | H |
| 443 | pentyl | H | H | 2-hydroxyethoxy | H |
| 444 | 1-ethylpropyl | H | H | 2-hydroxyethoxy | H |
| 445 | hexyl | H | H | 2-hydroxyethoxy | H |
| 446 | 2-methylpentyl | H | H | 2-hydroxyethoxy | H |
| 447 | heptyl | H | H | 2-hydroxyethoxy | H |
| 448 | 1-ethylpentyl | H | H | 2-hydroxyethoxy | H |
| 449 | 4-methylpentyl | H | H | 2-hydroxyethoxy | H |
| 450 | 4-ethylbutyl | H | H | 2-hydroxyethoxy | H |
| 451 | octyl | H | H | 2-hydroxyethoxy | H |
| 452 | 1-ethylhexyl | H | H | 1-hydroxymethoxy | H |
| 453 | decyl | H | H | 1-hydroxymethoxy | H |
| 454 | vinyl | H | H | 1-hydroxymethoxy | H |
| 455 | 1-propenyl | H | H | 1-hydroxymethoxy | H |

**Table 14**

| Compound No. | R¹ | R² | R³ | R⁴ | R⁵ |
|---|---|---|---|---|---|
| 456 | 2-butenyl | H | H | 1-hydroxymethoxy | H |
| 457 | 1-hexenyl | H | H | 1-hydroxymethoxy | H |
| 458 | 1-octenyl | H | H | 1-hydroxymethoxy | H |
| 459 | 1-decenyl | H | H | 1-hydroxymethoxy | H |
| 460 | 3-methyl-2-butenyl | H | H | 1-hydroxymethoxy | H |
| 461 | geranyl | H | H | 1-hydroxymethoxy | H |
| 462 | prenyl | H | H | 1-hydroxymethoxy | H |
| 463 | methyl | H | H | 3-hydroxypropoxy | H |
| 464 | ethyl | H | H | 3-hydroxypropoxy | H |
| 465 | butyl | H | H | 3-hydroxypropoxy | H |
| 466 | hexyl | H | H | 3-hydroxypropoxy | H |
| 467 | 2-methylpentyl | H | H | 3-hydroxypropoxy | H |
| 468 | octyl | H | H | 3-hydroxypropoxy | H |
| 469 | decyl | H | H | 3-hydroxypropoxy | H |
| 470 | 1-propenyl | H | H | 3-hydroxypropoxy | H |
| 471 | 1-octenyl | H | H | 3-hydroxypropoxy | H |
| 472 | geranyl | H | H | 3-hydroxypropoxy | H |
| 473 | ethyl | H | H | 4-hydroxybutoxy | H |
| 474 | butyl | H | H | 4-hydroxybutoxy | H |
| 475 | s-butyl | H | H | 4-hydroxybutoxy | H |
| 476 | hexyl | H | H | 4-hydroxybutoxy | H |
| 477 | 1-ethylpentyl | H | H | 4-hydroxybutoxy | H |
| 478 | octyl | H | H | 4-hydroxybutoxy | H |
| 479 | 2-butenyl | H | H | 4-hydroxybutoxy | H |
| 480 | prenyl | H | H | 4-hydroxybutoxy | H |
| 481 | ethyl | H | H | 2,3-dihydroxypropoxy | H |
| 482 | butyl | H | H | 2,3-dihydroxypropoxy | H |
| 483 | hexyl | H | H | 2,3-dihydroxypropoxy | H |
| 484 | octyl | H | H | 2,3-dihydroxypropoxy | H |
| 485 | decyl | H | H | 2,3-dihydroxypropoxy | H |
| 486 | 1-hexenyl | H | H | 2,3-dihydroxypropoxy | H |
| 487 | 3-methyl-2-butenyl | H | H | 2,3-dihydroxypropoxy | H |
| 488 | methyl | H | H | 3,4-dihydroxybutoxy | H |
| 489 | ethyl | H | H | 3,4-dihydroxybutoxy | H |
| 490 | hexyl | H | H | 3,4-dihydroxybutoxy | H |

**Table 15**

| Compound No. | R¹ | R² | R³ | R⁴ | R⁵ |
|---|---|---|---|---|---|
| 491 | octyl | H | H | 3,4-dihydroxybutoxy | H |
| 492 | 1-propenyl | H | H | 3,4-dihydroxybutoxy | H |
| 493 | 1-octenyl | H | H | 3,4-dihydroxybutoxy | H |
| 494 | geranyl | H | H | 3,4-dihydroxybutoxy | H |
| 495 | methyl | H | H | carboxymethoxy | H |
| 496 | ethyl | H | H | carboxymethoxy | H |
| 497 | propyl | H | H | carboxymethoxy | H |
| 498 | isopropyl | H | H | carboxymethoxy | H |
| 499 | butyl | H | H | carboxymethoxy | H |
| 500 | s-butyl | H | H | carboxymethoxy | H |
| 501 | pentyl | H | H | carboxymethoxy | H |
| 502 | 1-ethylpropyl | H | H | carboxymethoxy | H |
| 503 | hexyl | H | H | carboxymethoxy | H |
| 504 | 2-methylpentyl | H | H | carboxymethoxy | H |
| 505 | heptyl | H | H | carboxymethoxy | H |
| 506 | 1-ethylpentyl | H | H | carboxymethoxy | H |
| 507 | 4-methylpentyl | H | H | carboxymethoxy | H |
| 508 | 1-ethylhexyl | H | H | carboxymethoxy | H |
| 509 | octyl | H | H | carboxymethoxy | H |
| 510 | 1-ethylhexyl | H | H | carboxymethoxy | H |
| 511 | decyl | H | H | carboxymethoxy | H |
| 512 | vinyl | H | H | carboxymethoxy | H |
| 513 | 1-propenyl | H | H | carboxymethoxy | H |
| 514 | 2-butenyl | H | H | carboxymethoxy | H |
| 515 | 1-hexenyl | H | H | carboxymethoxy | H |
| 516 | 1-octenyl | H | H | carboxymethoxy | H |
| 517 | 1-decenyl | H | H | carboxymethoxy | H |
| 518 | 3-methyl2-butenyl | H | H | carboxymethoxy | H |
| 519 | geranyl | H | H | carboxymethoxy | H |
| 520 | prenyl | H | H | carboxymethoxy | H |
| 521 | methyl | H | H | 2-carboxyethoxy | H |
| 522 | ethyl | H | H | 2-carboxyethoxy | H |
| 523 | butyl | H | H | 2-carboxyethoxy | H |
| 524 | hexyl | H | H | 2-carboxyethoxy | H |
| 525 | octyl | H | H | 2-carboxyethoxy | H |

**Table 16**

| Compound No. | R¹ | R² | R³ | R⁴ | R⁵ |
|---|---|---|---|---|---|
| 526 | 1-propenyl | H | H | 2-carboxyethoxy | H |
| 527 | 1-octenyl | H | H | 2-carboxyethoxy | H |
| 528 | geranyl | H | H | 2-carboxyethoxy | H |
| 529 | ethyl | H | H | 3-carboxypropoxy | H |
| 530 | butyl | H | H | 3-carboxypropoxy | H |
| 531 | hexyl | H | H | 3-carboxypropoxy | H |
| 532 | octyl | H | H | 3-carboxypropoxy | H |
| 533 | 2-butenyl | H | H | 3-carboxypropoxy | H |
| 534 | prenyl | H | H | 3-carboxypropoxy | H |
| 535 | ethyl | H | H | 4-carboxybutoxy | H |
| 536 | butyl | H | H | 4-carboxybutoxy | H |
| 537 | hexyl | H | H | 4-carboxybutoxy | H |
| 538 | octyl | H | H | 4-carboxybutoxy | H |
| 539 | 1-octenyl | H | H | 4-carboxybutoxy | H |
| 540 | methyl | H | H | H | 2-hydroxyethoxy |
| 541 | ethyl | H | H | H | 2-hydroxyethoxy |
| 542 | propyl | H | H | H | 2-hydroxyethoxy |
| 543 | isopropyl | H | H | H | 2-hydroxyethoxy |
| 544 | butyl | H | H | H | 2-hydroxyethoxy |
| 545 | s-butyl | H | H | H | 2-hydroxyethoxy |
| 546 | pentyl | H | H | H | 2-hydroxyethoxy |
| 547 | hexyl | H | H | H | 2-hydroxyethoxy |
| 548 | 2-methylpentyl | H | H | H | 2-hydroxyethoxy |
| 549 | heptyl | H | H | H | 2-hydroxyethoxy |
| 550 | 1-ethylpentyl | H | H | H | 2-hydroxyethoxy |
| 551 | 4-methylpentyl | H | H | H | 2-hydroxyethoxy |
| 552 | 1-ethylhexyl | H | H | H | 2-hydroxyethoxy |
| 553 | octyl | H | H | H | 2-hydroxyethoxy |
| 554 | 1-ethylhexyl | H | H | H | 1-hydroxymethoxy |
| 555 | decyl | H | H | H | 1-hydroxymethoxy |
| 556 | vinyl | H | H | H | 1-hydroxymethoxy |
| 557 | 1-propenyl | H | H | H | 1-hydroxymethoxy |
| 558 | 2-butenyl | H | H | H | 1-hydroxymethoxy |
| 559 | 1-hexenyl | H | H | H | 1-hydroxymethoxy |
| 560 | 1-octenyl | H | H | H | 1-hydroxymethoxy |

**Table 17**

| Compound No. | R¹ | R² | R³ | R⁴ | R⁵ |
|---|---|---|---|---|---|
| 561 | 1-decenyl | H | H | H | 1-hydroxymethoxy |
| 562 | 3-methyl-2-butenyl | H | H | H | 1-hydroxymethoxy |
| 563 | geranyl | H | H | H | 1-hydroxymethoxy |
| 564 | prenyl | H | H | H | 1-hydroxymethoxy |
| 565 | methyl | H | H | H | 3-hydroxypropoxy |
| 566 | ethyl | H | H | H | 3-hydroxypropoxy |
| 567 | butyl | H | H | H | 3-hydroxypropoxy |
| 568 | hexyl | H | H | H | 3-hydroxypropoxy |
| 569 | 2-methylpentyl | H | H | H | 3-hydroxypropoxy |
| 570 | octyl | H | H | H | 3-hydroxypropoxy |
| 571 | decyl | H | H | H | 3-hydroxypropoxy |
| 572 | 1-propenyl | H | H | H | 3-hydroxypropoxy |
| 573 | 1-octenyl | H | H | H | 3-hydroxypropoxy |
| 574 | geranyl | H | H | H | 3-hydroxypropoxy |
| 575 | ethyl | H | H | H | 4-hydroxybutoxy |
| 576 | butyl | H | H | H | 4-hydroxybutoxy |
| 577 | s-butyl | H | H | H | 4-hydroxybutoxy |
| 578 | hexyl | H | H | H | 4-hydroxybutoxy |
| 579 | 1-ethylpentyl | H | H | H | 4-hydroxybutoxy |
| 580 | octyl | H | H | H | 4-hydroxybutoxy |
| 581 | 2-butenyl | H | H | H | 4-hydroxybutoxy |
| 582 | prenyl | H | H | H | 4-hydroxybutoxy |
| 583 | ethyl | H | H | H | 2,3-dihydroxypropoxy |
| 584 | butyl | H | H | H | 2,3-dihydroxypropoxy |
| 585 | hexyl | H | H | H | 2,3-dihydroxypropoxy |
| 586 | octyl | H | H | H | 2,3-dihydroxypropoxy |
| 587 | decyl | H | H | H | 2,3-dihydroxypropoxy |
| 588 | 1-hexenyl | H | H | H | 2,3-dihydroxypropoxy |
| 589 | 3-methyl-2-butenyl | H | H | H | 2,3-dihydroxypropoxy |
| 590 | methyl | H | H | H | 3,4-dihydroxybutoxy |
| 591 | ethyl | H | H | H | 3,4-dihydroxybutoxy |
| 592 | hexyl | H | H | H | 3,4-dihydroxybutoxy |
| 593 | octyl | H | H | H | 3,4-dihydroxybutoxy |
| 594 | 1-propenyl | H | H | H | 3,4-dihydroxybutoxy |
| 595 | 1-octenyl | H | H | H | 3,4-dihydroxybutoxy |

**Table 18**

| Compound No. | R¹ | R² | R³ | R⁴ | R⁵ |
|---|---|---|---|---|---|
| 596 | geranyl | H | H | H | 3,4-dihydroxybutoxy |
| 597 | methyl | H | H | H | carboxymethoxy |
| 598 | ethyl | H | H | H | carboxymethoxy |
| 599 | propyl | H | H | H | carboxymethoxy |
| 600 | isopropyl | H | H | H | carboxymethoxy |
| 601 | butyl | H | H | H | carboxymethoxy |
| 602 | s-butyl | H | H | H | carboxymethoxy |
| 603 | pentyl | H | H | H | carboxymethoxy |
| 604 | hexyl | H | H | H | carboxymethoxy |
| 605 | 2-methylpentyl | H | H | H | carboxymethoxy |
| 606 | heptyl | H | H | H | carboxymethoxy |
| 607 | 1-ethylpentyl | H | H | H | carboxymethoxy |
| 608 | 4-methylpentyl | H | H | H | carboxymethoxy |
| 609 | 1-ethylhexyl | H | H | H | carboxymethoxy |
| 610 | octyl | H | H | H | carboxymethoxy |
| 611 | 1-ethylhexyl | H | H | H | carboxymethoxy |
| 612 | decyl | H | H | H | carboxymethoxy |
| 613 | vinyl | H | H | H | carboxymethoxy |
| 614 | 1-propenyl | H | H | H | carboxymethoxy |
| 615 | 2-butenyl | H | H | H | carboxymethoxy |
| 616 | 1-hexenyl | H | H | H | carboxymethoxy |
| 617 | 1-octenyl | H | H | H | carboxymethoxy |
| 618 | 1-decenyl | H | H | H | carboxymethoxy |
| 619 | 3-methyl-2-butenyl | H | H | H | carboxymethoxy |
| 620 | geranyl | H | H | H | carboxymethoxy |
| 621 | prenyl | H | H | H | carboxymethoxy |
| 622 | methyl | H | H | H | 2-carboxyethoxy |
| 623 | ethyl | H | H | H | 2-carboxyethoxy |
| 624 | butyl | H | H | H | 2-carboxyethoxy |
| 625 | hexyl | H | H | H | 2-carboxyethoxy |
| 626 | octyl | H | H | H | 2-carboxyethoxy |
| 627 | 1-propenyl | H | H | H | 2-carboxyethoxy |
| 628 | 1-octenyl | H | H | H | 2-carboxyethoxy |
| 629 | geranyl | H | H | H | 2-carboxyethoxy |
| 630 | ethyl | H | H | H | 3-carboxypropoxy |

**Table 19**

| Compound No. | R¹ | R² | R³ | R⁴ | R⁵ |
|---|---|---|---|---|---|
| 631 | butyl | H | H | H | 3-carboxypropoxy |
| 632 | hexyl | H | H | H | 3-carboxypropoxy |
| 633 | octyl | H | H | H | 3-carboxypropoxy |
| 634 | 2-butenyl | H | H | H | 3-carboxypropoxy |
| 635 | prenyl | H | H | H | 3-carboxypropoxy |
| 636 | ethyl | H | H | H | 4-carboxybutoxy |
| 637 | butyl | H | H | H | 4-carboxybutoxy |
| 638 | hexyl | H | H | H | 4-carboxybutoxy |
| 639 | octyl | H | H | H | 4-carboxybutoxy |
| 640 | 1-octenyl | H | H | H | 4-carboxybutoxy |

The term "physiologically acceptable salts" as used herein means nontoxic alkali addition salts of, for example, the above-described compounds, which include sodium salts, potassium salts, magnesium salts, calcium salts, ammonium salts, and the like. These physiologically acceptable salts can be produced by known methods from the benzopyran derivatives represented by the aforementioned general formula (I).

The benzopyran derivatives represented by the general formula (I) have excellent stability and bioabsorption compared to the aforementioned comparative compounds A, B and C disclosed in Journal of Medicinal Chemistry, volume 31, p.1437 to 1445, 1988 (Donald. T. Witiak, J. Med. Chem., Vol. 31, P.1437-1445, 1988.) (Non-patent Publication No.1) and US Patent No. 4845121 (Patent Publication No.3), as described later in examples. Therefore, the benzopyran derivatives represented by the general formula (I) are excellent active ingredients having favorable characteristics, especially when used as pharmaceutical agents.

Additionally, the benzopyran derivatives represented by the general formula (I) have low toxicity and excellent therapeutic effects on circulatory insufficiency, as described later in examples.

The term "circulatory insufficiency" as used herein includes occlusive or functional arterial diseases, venous diseases and complex arteriovenous diseases. For example, acute arterial occlusion, chronic arterial obstruction, functional circulatory disorder, and secondary circulatory disorders due to diabetes mellitus and the like.

The aforementioned acute arterial occlusion includes the acute thrombosis due to the rupture of proximal atherosclerotic plaques (i.e. a yellow atheromatous substance formed on the endothelial surface due to the lipid deposition in the endarterium and such an atheromatous substance may decrease or disrupt blood flow) or latent atherosclosis (i.e. arteriosclosis characterized by lipid depositon irregulary distributed in the intima of aorta or medium-sized artery). The acute occlusion also includes venous thrombosis, deep-venous thrombosis, pulmonary embolism or the like that can be developed in veins due to the similar mechanisms, and such a disease can be caused from thrombus that travels from the heart, aorta or other large-sized vessel. Additionally, the acute occlusion further includes thrombus, embolus and vascular stenosis that occur secondary to external injury, surgery, percutaneous transluminal coronary angioplasty (PTCA), coronary artery bypass graft surgery (CAGB) and the like.

The aforementioned chronic arterial occlusion, which presents chronic ischemia, is a disease developed and progressed due to gradual expansion of atheromatous plaques (i.e. a yellow limited area or swelling on the intimal surface of the artery due to the lipid desposition in the endomembrane). The chronic arterial occlusion also includes thromboangitis obliterans and Buerger's disease.

The aforementioned functional circulatory disorder includes vasospastic Raynaud's phenomenon, Raynaud's disease, acrocyanosis and the like. The aforementioned secondary circulatory disorder includes circulatory disorders that occur secondary to diseases such as diabetes mellitus, maintenance hemodialysis, collagen disease, hypertension, or hyperlipemia.

The benzopyran derivatives represented by the general formula (I) have soothing effects and therapeutic effects against numbness, coldness, intermittent claudication, pain at rest, ulcer, extremity ulcer, cutaneous ulcer, gangrene, among others, that accompany the above-mentioned diseases. Additionally, the benzopyran derivatives can be used for prophylactic purposes to prevent the onset and recurrence of cerebral infarction caused from thrombotic or embolic ischemic disorders.

The improving effect on circulatory insufficiency in the present invention is completely different from the anti-allergic effect or the therapeutic effect for heart diseases disclosed in Japanese Unexamined Patent Application, Publication No. 2003-81827 (Patent Publication No.1) or Japanese Unexamined Patent Application, Publication No. Hei 09-315967 (Patent Publication No.2). Namely, the anti-allergic effect described in Japanese Unexamined Patent Application, Publication No. 2003-81827 (Patent Publication No.1) is a preventive or therapeutic effect against allergic diseases caused by the excessively activated immune system in a living body induced by external or internal antigens. Such allergic diseases include, for example, immediate asthma, delayed asthma, bronchial asthma, pediatric asthma, nasal congestion, atopic dermatitis, allergic dermatitis, hives, eczema, allergic conjunctivitis, allergic rhinitis, pollenosis, food allergy, allergic gastroenteritis, allergic colitis, drug allergy, contact dermatitis and autoimmune diseases, and thus are completely different from circulatory insufficiency described in the present invention.

The heart diseases described in Japanese Unexamined Patent Application, Publication No. Hei 09-315967 (Patent publication No.2) include arrhythmia such as supraventricular extrasystole, paroxysmal supraventricular tachycardia, paroxysmal atrial fibrillation, chronic atrial fibrillation, atrial fibrillation, premature ventricular contraction, ventricular tachycardia, ventricular fibrillation and atrioventricular block, arrhythmia accompanied with ischemic cardiopathy (such as myocardial infarction and cardiac angina), acute myocardial infarction, chronic myocardial infarction, cardiac failure, cardiac angina and the like. Thus, these heart diseases are completely different from circulatory insufficiency described in the present invention.

The drug for treating circulatory insufficiency containing the benzopyran derivatives represented by the general formula (I) as active ingredients can be administered orally or parenterally (for example, intravenous administration, subcutaneous administration, percutaneous absorption, rectal administration or the like). Such a pharmaceutical agent can be made into various dosage forms according to the purpose, such as tablets, capsules, granules, fine subtilaes, powders, troches, sublingual tablets, suppositories, ointments, injections, emulsions, suspensions, medicated syrups, chewable tablets and the like.

These dosage forms can be prepared in accordance with known techniques using pharmaceutically-acceptable additives commonly used in these types of drugs, such as excipients, bonding agents, disintegrators, lubricants, preservatives, anti-oxidative agents, isotonic agents, buffering agents, coating agents, sweetening agents, solubilizing agents, bases, dispersing agents, stabilizing agents, coloring agents and the like. Illustrative examples of these pharmaceutically acceptable additives are listed in the following.

Firstly, as excipients, the following can be listed: starch and derivatives of starch (such as dextrin, or carboxymethyl starch), cellulose and derivatives of cellulose (such as methylcellulose, or hydroxypropylmethylcellulose), sugars (such as lactose, sucrose, or glucose), silicic acid and silicates (such as natural aluminum silicate, or magnesium silicate), carbonates (such as calcium carbonate, magnesium carbonate, sodium bicarbonate), aluminum magnesium hydroxide, synthetic hydrotalcite, polyoxyethylene derivatives, glyceryl monostearate, sorbitan monooleate and the like.

As bonding agents, the following can be listed: starch and starch derivatives (such as alpha starches, or dextrin), cellulose and derivatives of cellulose (such as ethyl cellulose, sodium carboxymethyl cellulose, or hydroxypropyl methylcellulose), gum arabic, traganth, gelatin, sugars (such as glucose, or sucrose), ethanol, polyvinyl alcohols and the like.

As disintegrators, the following can be listed: starch and starch derivatives (such as carboxymethyl starch, or hydroxypropyl starch), cellulose and cellulose derivatives (such as sodium carboxymethyl cellulose, crystalline cellulose, or hydroxypropyl methylcellulose), carbonates (such as calcium carbonate, or calcium bicarbonate), traganth, gelatin, agar and the like.

As lubricants, the following can be listed: stearic acid, calcium stearate, magnesium stearate, talc, silicic acid and its salts (such as light silicic anhydrides, or natural aluminum silicates), titanium oxide, calcium hydrogen phosphate, dry aluminum hydroxide gel, macrogol and the like.

As preservatives, the following can be listed: p-hydroxybenzoate esters, sulfites (such as sodium sulfites, or sodium pyrosulfite), phosphates (such as sodium phosphate, calcium polyphosphate, sodium polyphosphate, or sodium metaphosphate), alcohols (such as chlorobutanol, or benzyl alcohol), benzalkonium chloride, benzethonium chloride, phenol, cresol, chlorocresol, dihydroacetic acid, sodium dihydroacetate, glyceryl sorbate, sugars and the like.

As anti-oxidative agents, the following can be listed: sulfites (such as sodium sulfite, or sodium bisulfite), rongalite, erythorbic acid, L-ascorbic acid, cysteine, thioglycerol, butylhydroxyanisol, dibutylhydroxytoluene, propyl gallate, ascorbyl palmitate, dl-alpha-tocopherol and the like.

As isotonic agents, the following can be listed: sodium chloride, sodium nitrate, potassium nitrate, dextrin, glycerol, glucose and the like.

As buffering agents, the following can be listed: sodium carbonate, hydrochloric acid, boric acid, phosphates (such as sodium hydrogen phosphate) and the like.

As coating agents, the following can be listed: cellulose derivatives (such as hydroxypropyl cellulose, cellulose acetate phthalate, or hydroxypropyl methylcellulose phthalate), shellac, polyvinylpyrrolidone, polyvinylpyridines (such as poly-2-vinylpyridine, or poly-2-vinyl-5-ethylpyridine), polyvinylacetyl diethylaminoacetate, polyvinyl alcohol phthalate, methacrylate/methacrylate copolymers and the like.

As sweetening agents, the following can be listed: sugars (such as glucose, sucrose, or lactose), sodium saccharin, sugar alcohols and the like.

As solubilizing agents, the following can be listed: ethylenediamine, nicotinamide, sodium saccharin, citric acid, citrates, sodium benzoate, soaps, polyvinylpyrrolidone, polysorbate, sorbitan fatty acid esters, glycerol, propylene glycol, benzyl alcohols and the like.

As bases, the following can be listed: fats (such as lard), vegetable oils (such as olive oil, or sesame oil), animal oil, lanolin acid, petrolatums, paraffin, wax, resins, bentonite, glycerol, glycol oils, higher alcohols (such as stearyl alcohol, or cetanol) and the like.

As dispersing agents, the following can be listed: gum arabic, traganth, cellulose derivatives (such as methyl cellulose), stearic acid polyesters, sorbitan sesquioleate, aluminum monostearate, sodium alginate, polysorbate, sorbitan fatty acid esters and the like.

Lastly, as stabilizing agents, the following can be listed: sulfites (such as sodium bisulfite), nitrogen, carbon dioxide and the like.

Although the content of the benzopyran derivatives represented by the general formula (I) in these pharmaceutical preparations varies depending on the dosage forms, they may be contained preferably in a concentration of from 0.01 % to 100% by weight.

The dose of the drug for treating circulatory insufficiency of the present invention can be varied over a broad range depending on each warm-blooded animal to be treated, including humans, severity of the symptoms, doctor's judgement, among others. In general, however, it may be administered preferably in a dose of from 0.01 to 100 mg, more preferably from 0.1 to 70 mg, as the active ingredient, per day per kg body weight in the case of oral administration. In the same way, it may be administered preferably in a dose of from 0.01 to 100 mg, more preferably from 0.1 to 70 mg, as the active ingredient, per day per kg body weight in the case of parenteral administration. The daily dose described above may be administered once a day or divided into several batches, and may be also changed optionally in accordance with the extent of diseases and doctor's judgement.

### Examples

The present invention will be described in detail with reference to examples. However, the present invention is not limited to examples.

### (EXAMPLE 1) (Acute toxicity test in rats)

We performed this test using rats in order to confirm the safety of the benzopyran derivatives used in the present invention (to be referred to as "the compounds of the present invention" hereinafter).

### <Method>

The compounds of the present invention Nos. 9, 67, 98, 118, 119, 120, 121, 123, 124, 125, 131, 141, 144, 174, 179, 196, 214, 237, 244, 261, 280, 295, 333, 347, 388, 429, 445, 449, 451, 468, 477, 485, 491, 506, 525, 547, 551, and 633 were added to 0.5 (w/v) % methyl cellulose solution and prepared. Each solution was administered with oral gavage at the doses of 500, 1000 and 2000 mg/kg to male SD rats (body weight is 120 to 200 g, 5 rats per one group), using a feeding tube for rats.

After the administration, the animals were kept in cages for 7 days, to observe general symptoms and to count dead animals. Lethal dose (LD₅₀: mg/kg) was extrapolated from the mortality at the 7^{th} day after administration.

### <Result>

In the result, the LD₅₀ of all compounds tested were over 2000 mg/kg, and therefore it was clearly shown that the compounds of the present invention, the benzopyran derivatives, have high safety.

### (EXAMPLE 2) (The pharmacological effect on a circulatory insufficiency model induced by lauric-acid in rats)

We performed this test in order to evaluate the pharmacological effect of the compounds of the present invention using a circulatory insufficiency model of rats induced by injection of lauric-acid into their femoral artery.

### <Method>

13-week-old male Wistar rats (body weight is 280 to 316 g), 8 rats per one group, were used. The rats were held in a supine position under anesthesia due to administration of 40 mg/kg of sodium pentobarbital by intraperitoneal injection. Then, the right femoral area was incised, thereby injecting 0.15 mL of 10 mg/mL lauric-acid solution into the femoral artery in order to induce lower limb gangrene caused by the peripheral vascular disorder. A few drops of instant adhesive (Aron-alpha; registered trademark) were used to stop bleeding, followed by topical application of antibiotics (potassium penicillin G solution) to prevent infection, and the incision site was then sutured.

Each compound of the present invention was added to 0.5 (w/v) % methyl cellulose solution to prepare 0.5 (w/v) % methyl cellulose suspension contaning the compound of the present invention. The suspension was administered, by means of multiple oral dosing, 1 hour prior to and 3 hours after injection of lauric-acid and twice daily (at 10:00 and 17:00) for the following 9 days, at the dose of 30 mg/kg for each compound. Ticlopidine hydrochloride was added to 0.5 (w/v) % methyl cellulose solution to prepare 0.5 (w/v) % methyl cellulose suspension contaning ticlopidine hydrochloride to use as a positive control. The suspension was administered orally 3 hours prior to injection of lauric-acid at the dose of 300 mg/kg.

The extent of lesions was evaluated 3 days and 10 days after injection of lauric acid by the following criteria:

| | <Point> |
|---|---|
| No change (Normal) | : 0 |
| Black discoloration limited to tiptoes | : 1 |
| Black discoloration of toes | : 2 |
| Necrosis of toes | : 3 |
| Loss of toes | : 4 |

The lesion of each toe was graded and the total points of 5 toes were use as a lesion index, wherein 5 points were further added when the lesion reached the heel (i.e. the maximum lesion index was 25 points).

### <Results>

The pharmacological effects of the control (vehicle treatment) group and each compound are shown in Table 20. The shown number refers to the average value of the lesion index obtained from the evaluation.

**Table 20**

| The pharmacological effect on a lauric-acid-induced model | | | | | |
|---|---|---|---|---|---|
| Compound No. | Lesion Index | | Compound No. | Lesion Index | |
| | after 3 days | after 10 days | | after 3 days | after 10 days |
| 9 | 2.5 | 7.8 | 280 | 2.7 | 7.9 |
| 67 | 2.8 | 7.5 | 295 | 3.0 | 7.2 |
| 98 | 3.0 | 8.4 | 333 | 3.0 | 7.4 |
| 118 | 2.4 | 8.3 | 347 | 2.3 | 8.1 |
| 119 | 2.5 | 7.1 | 388 | 2.9 | 7.5 |
| 120 | 2.3 | 8.0 | 429 | 2.2 | 7.9 |
| 121 | 2.8 | 7.3 | 445 | 2.7 | 7.5 |
| 123 | 2.6 | 7.9 | 449 | 2.8 | 7.3 |
| 124 | 3.0 | 8.3 | 451 | 2.6 | 7.2 |
| 125 | 2.6 | 8.1 | 468 | 2.8 | 7.8 |
| 131 | 3.0 | 7.5 | 477 | 2.9 | 7.7 |
| 141 | 2.6 | 7.8 | 485 | 2.8 | 7.9 |
| 144 | 2.5 | 7.2 | 491 | 2.9 | 7.4 |
| 174 | 2.7 | 7.5 | 506 | 2.7 | 7.3 |
| 179 | 3.6 | 8.4 | 525 | 2.9 | 7.8 |
| 196 | 3.3 | 7.4 | 547 | 3.0 | 7.2 |
| 214 | 3.5 | 7.9 | 551 | 2.5 | 7.8 |
| 237 | 3.4 | 7.9 | 633 | 2.6 | 7.4 |
| 244 | 3.5 | 7.4 | control | 7.5 | 19.4 |
| 261 | 3.7 | 7.6 | Ticlopidine hydrochloride | 2.8 | 7.4 |

The results clearly showed that the compounds of the present invention decreased the lesion index compared to the control (vehicle treatment) group. This revealed that their pharmacological effect was equal to or greater than that of the positive control of ticlopdine hydrochloride. Thus, it was evident that the compounds of the present invention were useful as a drug for treating circulatory insufficiency.

### (EXAMPLE 3) (Effect on bleeding time in rats)

### <Method>

5-week-old male SD rats (body weight is 138 to 152 g), 6 rats per one group, were used. The comparative substances (aspirin, cilostazol, beraprost sodium and ticlopdine hydrochloride) or the coumpounds of the present invention (compound Nos. 125, 144, 445, 451 and 525) were added to 0.5 (w/v) % methyl cellulose solution to prepare 0.5 (w/v) % methyl cellulose suspensions containing the comparative substances or the compounds of the present invention. The suspension was administered orally at the doses of 100 mg/kg for aspirin, 300 mg/kg for cilostazol, 1 mg/kg for beraprost sodium and 30 mg/kg for each compound of the present invention (compound No. 125, 144, 445, 451 and 525). 50 minutes after the administration, 50 mg/kg of pentobarbital sodium was intraperitoneally injected into the rat.

Because the pharmacologically-active form of ticlopidine hydrochloride (comparative substrance) is its *in vivo* metabolite, the time between the administration of the test compound and tail cutting was set longer. Namely, 2 hours and 50 minutes after the administration of ticlopidine hydrochloride, 50 mg/kg of pentobarbital sodium was injected intraperitoneally. 10 minutes later, the tail was cut off at a position of 2 mm from the tip using a surgical blade, and was immediately immersed into a glass container (Magnus bath) filled with physiological saline maintained at approximately 37°C to observe until the rat stopped bleeding.

The bleeding time was taken as the time from the tail cutting to the cessation of bleeding. The tail was marked at a position of 5 cm from the tip in advance, and was immersed in the physiological saline in the glass container at the depth of 5 cm from the surface. The maximum observation time was defined as 60 minutes after the tail cutting.

### <Results>

Table 21 shows the results of those having 60 minutes between oral dosing of vehicle (control) or test compounds and the tail cutting

**Table 21**

| Compounds | Dosage (mg/kg) | Time (min) between oral dosing and the tail cutting | Bleeding time (min.) |
|---|---|---|---|
| vehicle | - | 60 | 4.9 |
| aspirin | 100 | 60 | 40.2 |
| cilostazol | 300 | 60 | 50.1 |
| beraprost sodium | 1 | 60 | 42.2 |
| 125 | 30 | 60 | 8.0 |
| 144 | 30 | 60 | 8.9 |
| 445 | 30 | 60 | 9.1 |
| 451 | 30 | 60 | 7.8 |
| 525 | 30 | 60 | 8.5 |

Table 22 shows the results of those having 180 minutes between oral dosing of vehicle (control) or test compounds including ticlopidine hydrochloride and the tail cutting.

**Table 22**

| Compounds | Dosage (mg/kg) | Time (min) between oral dosing and the tail cutting | Bleeding time (min.) |
|---|---|---|---|
| vehicle | - | 180 | 6.3 |
| ticlopidine hydrochloride | 300 | 180 | 36.1 |
| 125 | 30 | 180 | 8.4 |
| 144 | 30 | 180 | 8.2 |
| 445 | 30 | 180 | 8.3 |
| 451 | 30 | 180 | 7.5 |
| 525 | 30 | 180 | 8.1 |

The results clearly showed that the compounds of the present invention were drugs having little effect on bleeding time prolongation compared with the existing drugs.

### (EXAMPLE 4) (Stability)

In order to demonstrate stability of the compounds of the present invention, a stability test was conducted in an acidic solution or basic solution with respect to the compounds No.125, 451 and 525 of the present invention, and the comparative compounds A, B and C.

### <Method>

The test compounds were dissolved in an acidic solution (phosphate buffer (pH3.4)) and in a basic solution (phosphate buffer (pH7.3)) at the concentration of 1 mmoL/L. Immediately after they were dissolved, each solution was analyzed with liquid chromatography using an acidic solution (phosphate buffer (pH3.4)) or basic solution (phosphate buffer (pH7.3)) as an eluent. The peak area of the test compounds was measured as the initial value. Furthermore, a time-course analysis with liquid chromatograpy was conducted to measure the peak area at each time point. The solution containing the test compound was kept in an incubator at 37°C. Based on the measured peak area of the test compounds, the percentage (%) of the peak area at each measurement time point was calculated, taking the peak area of the initial value as 100 (%). The half-life (the time to show a 50% residual rate of the test compounds) of the test compounds was further calculated, and its stability was evaluated based on the half-life.

### <Result>

The results of the present example are shown in Table 23.

**Table 23**

| Compounds | Half-life (hr) | |
|---|---|---|
| | pH 3.4 solution | pH 7.3 solution |
| Comparative compound A | 30 | 65 |
| Comparative compound B | 53 | 70 |
| Comparative compound C | 35 | 65 |
| 125 | >>100 | >>100 |
| 451 | >>100 | >>100 |
| 525 | >>100 | >>100 |

In this result, no decrease in peak area was observed even after 100 hours, and this revealed that no degradation occurred with regard to the compounds of the present invention. Thus, the results clearly demonstrated that the compounds of the present invention were superior in stability to the aforementioned comparative compound A, B or C disclosed in Journal of Medicinal Chemistry, volume 31, p.1437 to 1445, 1988 (Donald.T.Witiak, J.Med.Chem., Vo L .31,P.1437-1445,1988.) (Non-patent publication No.1) and US Patent No. 4845121 (Patent Publication No.3) .

### (EXAMPLE 5) (Bioabsorption)

We conducted a plasma concentration measurement in rats with oral administration to compare the absorption of the compound of the present invention (compound No.451) with the comparative compound A and B.

### <Method>

### 1. Administration and blood sampling

6-week-old male SD rats (body weight is 200 to 230 g) were used for this test (3 rats per one group). The required amount of test compound was weighed and pulverized in an agate mortar. Then, a 0.5 (w/v) % methyl cellulose solution was added to prepare the suspension at the concentration of 10 mg/5mL. 5 mL per kg body weight of the suspension was orally administered to rats once using a feeding tube for rats. About 0.3 mL of blood was sampled from the caudal vein using a heparinized glass tube at 0.25, 0.5, 1, 2, 4, 6, 8, 12 and 24 hours after the administration, and was centrifuged to obtain plasma.

### 2. Sample preparation

30 µL of methanol and 300 µL of acetonitrile were added to 120 µL of the obtained plasma, and mixed with a Vortex mixer for 15 seconds. The sample was centrifuged to obtain 300 µL of the supernatant. The supernatant was dried, and 120 µL of an eluent was added thereto, and then mixed with a Vortex mixer for 15 seconds. After the mixture was centrifuged, the amount of each test compound remaining in the plasma was determined with liquid chromatography.

### 3. Measurement determination of the test compound with liquid chromatography

40 µL of the sample, as prepared above, was applied to liquid chromatography with the following conditions to conduct the measurement.
(1) Liquid chromatographic condition for the compound No.451
   Column: InertsiL (registered trademark) ODS-3 4.6 mmI.D. x 250 mm;
   Column temperature: 40°C;
   Eluent: Solution A (10 mmoL/L ammonium acetate : methanol = 50 : 50), and Solution B (10 mmoL/L ammonium acetate : methanol = 10 : 90);
   Gradient condition (eluent composition): solution A → 20 min. → solution B (5 min. elution) → 1 min. → solution A (12 min. elution);
   Flow rate: 1.0 mL; and
   Detection wavelength: 314 nm.
(2) Liquid chromatographic condition for the comparative compound A
   Column: InertsiL (registered trademark) ODS-3 4.6 mmI.D. x 150 mm;
   Column temperature: 40°C;
   Eluent: Solution A (aqua : methanol : acetic acid = 90 : 10 : 0.5), and Solution B (aqua :
   methanol : acetic acid = 10 : 90 : 0.5);
   Gradient condition (eluent composition): solution A → 20 min. → solution B (5 min. elution) → 1 min. → solution A (12 min. elution);
   Flow rate: 1.0 mL; and
   Detection wavelength: 323 nm.
(3) Liquid chromatographic condition for the comparative compound B Column: InertsiL (registered trademark) ODS-3 4.6 mmI.D. x 150 mm;
   Column temperature: 40°C;
   Eluent: Solution A (pH = 2.2 phosphate buffer : acetonitrile = 90 : 10), and Solution B (pH = 2.2 phosphate buffer : acetonitrile = 10:90);
   Gradient condition (eluent composition): Solution A → 10 min. → Solution B (2.5 min. elution) → 0.5 min. → Solution A (9 min. elution);
   Flow rate: 2.0 mL; and
   Detection wavelength: 315nm.

### (Result)

Based on the peak area obtained in the liquid chromatography analysis with respect to 1 µg/mL of each test compound in plasma, the plasma concentration (µg/mL) at each time point was calculated. The results are presented in Table 24.

**Table 24**

| Time point (h) | Compound No.451 | | Comparative Compound A | | Comparative Compound B | |
|---|---|---|---|---|---|---|
| | Mean | S.D. | Mean | S.D. | Mean | S.D. |
| 0.25 | 23.6 | 13.6 | 0.6 | 0.1 | 7.4 | 0.0 |
| 0.5 | 23.6 | 11.2 | 0.3 | 0.0 | 4.1 | 1.0 |
| 1.0 | 17.9 | 10.3 | 0.3 | 0.0 | 2.0 | 0.4 |
| 2.0 | 11.8 | 9.9 | 0.3 | 0.0 | 1.4 | 0.9 |
| 4.0 | 9.2 | 4.9 | 0.3 | 0.0 | 0.3 | 0.2 |
| 6.0 | 6.8 | 3.7 | 0.3 | 0.0 | 0.1 | 0.1 |
| 8.0 | 3.0 | 2.5 | 0.2 | 0.0 | 0.1 | 0.0 |
| 12.0 | 1.2 | 1.2 | Not detected | - | 0.0 | 0.0 |
| 24.0 | 0.5 | 0.4 | Not detected | - | 0.0 | 0.0 |

Additionally, each pharmacokinetic parameter calculated from the results is presented in Table 25, where "Cₘₐₓ" refers to the maximum plasma concentration, "Tₘₐₓ" refers to the time required to reach to the maximum plasma concentration, and "AUC" refers to the area under the plasma concentration-time curve, which represents the sum of the plasma concentration observed from the time point of administration of each test compound to the time point of 24 hours after the administration.

**Table 25**

| | Compound No.451 | Comparative Compound A | Comparative Compound B |
|---|---|---|---|
| Cₘₐₓ (µg/mL) | 25.1±13.3 | 0.6±0.1 | 7.4±0.2 |
| Tₘₐₓ (h) | 0.3±0.1 | 0.25±0.0 | 0.25±0.0 |
| AUC (µg·h/mL) | 99.5±64.4 | 2.2±0.1 | 8.3±2.6 |

In the results, it was clearly shown that the plasma concentration of the compounds of the present invention was about 3 to 40 times higher than that of the comparative compound A or B at each time point, and that such a high concentration can be maintained for a long time in plasma. Thus, it was evident that the compounds of the present invention were compounds having excellent bioabsorption.

It was clearly shown from the results of Examples 4 and 5 that the compounds of the present invention were superior in stability and bioabsorption compared to the comparative compounds A, B or C disclosed in Journal of Medicinal Chemistry, volume 31, p.1437 to 1445, 1988 (Dona L d.T. Witiak, J.Med.Chem., VoL. 31, P.1437-1445, 1988.) (Non-patent Publication No.1) and US Patent No. 4845121 (Patent Publication No.3). Accordingly, the compounds of the present invention have excellent characteristics to be used as pharmaceutical agents.

### (EXAMPLE 6) (100 mg tablet)

To produce a 100 mg tablet, 100 mg of compound No. 451, 50 mg of lactose, 20 mg of crystalline cellulose, 20 mg of crosscarmellose sodium, 9 mg of hydroxypropyl cellulose and 1 mg of magnesium stearate (i.e. total of 200 mg/tablet) were used (750-fold volume of each component was actually used to produce the 100 mg tablet, as described below).

First, compound No.451 was pulverized with a jet mill to obtain its pulverized powder. Next, 37.5 g of lactose, 15 g of crystalline cellulose, 15 g of crosscarmellose sodium and 75 g of the pulverized power of compound No.451 were mixed in the granulator. Then, the mixture was granulated while spraying 67.5 g of a 10% hydroxypropy cellulose solution. After drying, 0.75 g of magnesium stearate was added to the resulting mixture, and the mixture was pulverized in a cutter mill, and further mixed. Then, the mixture was loaded into a tableting machine to obtain objective tablets.

### (EXAMPLE 7) (10% powders)

100 mg of the compound 451 crystals was pulverized with a mortar, and 900 mg of lactose was added thereto. The mixture was thoroughly mixed by way of pulverizing with a pestle to obtain 10% powders.

### (EXAMPLE 8) (10% granules)

300 mg of the compound 525 was mixed with 300 mg of starch in a mortar, and the mixture was pulverized therein. This was further mixed with 2000 mg of lactose and 370 mg of starch. Separately from this, 30 mg of gelatin was mixed with 1 mL of purified water, solubilized by heating, and cooled. Then, 1 mL of ethanol was added thereto while stirring whereby a gelatin solution was prepared. Thereafter, the above-prepared mixture was mixed with the gelatin solution, and the resulting mixture was kneaded, granulated and then, dried to obtain granules.

### INDUSTRIAL APPLICABILITY

The drug containing as an active ingredient the benzopyran derivatives of the present invention can be medically applicable as a therapeutic agent for circulatory insufficiency. Additionally, the use of the aforementioned drug of the present invention and the method for treating circulatory insufficiency using the aforementioned drug of the present invention can be medically applicable for circulatory insufficiency because of their remarkable effectiveness in treating circulatory insufficiency.

## Claims

1. A drug for treating circulatory insufficiency containing a benzopyran derivative represented by the following general formula (I): 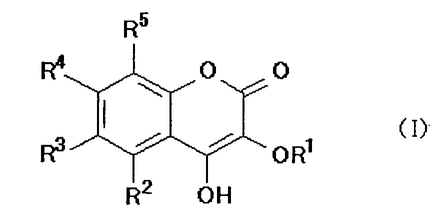 and/or a physiologically acceptable salt thereof as an active ingredient,
wherein R¹ is an alkyl group having 1 to 10 carbon atoms, or an alkenyl group having 2 to 10 carbon atoms; and any one of R², R³, R⁴ and R⁵ is a hydroxyl group, an alkoxy group, an alkenyloxy group, an alkoxy group substituted with a hydroxyl group, or an alkoxy group substituted with a carboxy group, and the others are hydrogen atoms.

2. The drug for treating circulatory insufficiency according to Claim 1, wherein R¹ is an alkyl group having 1 to 10 carbon atoms or an alkenyl group having 2 to 10 carbon atoms; and any one of R², R³, R⁴ and R⁵ is a hydroxyl group, an alkoxy group having 1 to 10 carbon atoms, an alkenyloxy group having 2 to 10 carbon atoms, an alkoxy group having 1 to 4 carbon atoms substituted with an hydroxyl group, or an alkoxy group having 1 to 4 carbon atoms substituted with an carboxy group, and the others are hydrogen atoms.

3. The drug for treating circulatory insufficiency according to Claim 1 or 2,
wherein the alkoxy group substituted with a hydroxyl group is an alkoxy group substituted with 1 or 2 hydroxyl groups.

4. Use of the drug for treating circulatory insufficiency according to any one of Claims 1 to 3 for treating circulatory insufficiency.

5. The method for treating circulatory insufficiency, the method comprising: using the drug for treating circulatory insufficiency according to any one of Claims 1 to 3.
